# EUROPÄISCHE PATENTSCHRIFT

(11) **EP 2 949 294 B1**
(45) Veröffentlichungstag und Bekanntmachung des Hinweises auf die Patenterteilung: **13.02.2019**
(21) Anmeldenummer: 15168896.7
(22) Anmeldetag: 22.05.2015
(51) Int. Cl.: A61F 2/80

(54) **PROTHESE ODER ORTHESE MIT HERAUSNEHMBAREM WANDSTÜCK**
PROSTHESIS OR ORTHESIS WITH REMOVABLE WALL PIECE
PROTHÈSE OU ORTHÈSE DOTÉE D'UN ÉLÉMENT DE PAROI AMOVIBLE

(30) Priorität: 26.05.2014 DE 202014004380 U
(43) Veröffentlichungstag der Anmeldung: 02.12.2015
(73) Patentinhaber: Pohlig GmbH, 83278 Traunstein (DE)
(72) Erfinder: Schäfer, Michael, 83278 Traunstein (DE); Pohlig, Kurt, 83289 Traunstein (DE)
(74) Vertreter: Pfenning, Meinig & Partner mbB

(56) Entgegenhaltungen:
- DE-U1- 9 419 211
- DE-U1-202006 007 460
- US-A- 2 533 404
- US-A1- 2004 260 402
- US-A1- 2014 025 183

## Beschreibung

Die Erfindung betrifft eine Prothese oder Orthese für den Ersatz von äußeren Körperteilen oder Gliedmaßen, beispielsweise nach Amputationen, bzw. zum Stabilisieren, Entlasten, Ruhigstellen und Führen von Gliedmaßen oder des Rumpfs oder zum Korrigieren von Fehlstellungen von Gliedmaßen oder des Rumpfs.

Bei den im Stand der Technik bekannten Prothesen oder Orthesen ist eine Behandlung eines Amputationsstumpfes oder einer mit einer Orthese therapierten Gliedmaße meist nur nach Abnehmen der Prothese oder Orthese, z.B. zur Verabreichung eines Medikaments in das Körpergewebe, möglich. Ist die Prothese oder Orthese an den Amputationsstumpf oder das zu therapierende Körperteil angelegt, überdecken der Prothesenschaft oder die Schalenteile der Orthese den Amputationsstumpf bzw. das Körperteil großflächig, so dass der Stumpf bzw. das Körperteil nachträglich nicht lokal wählbar mit Medikamenten versorgt werden kann.

Ferner sind im Stand der Technik Prothesen oder Orthesen bekannt, die über die oben genannten Funktionen, wie den Ersatz oder das Ruhigstellen von Gliedmaßen, hinaus weitere Funktionen besitzen. Beispielsweise ist es möglich in einen Prothesenschaft Kammern zu integrieren, in welchen Medikamentendepots zur Abgabe von Medikamenten an einen Amputationsstumpf eingesetzt sind. Des Weiteren sind Prothesen oder Orthesen bekannt, die im Prothesenschaft angeordnete Heizelemente zur Erwärmung eines Amputationsstumpfes oder Druckelemente zur Stimulation von Akupressurpunkten aufweisen. Andere Prothesen oder Orthesen beinhalten wiederum Sensoren zur Überwachung von Körperzuständen, wie zum Beispiel des Blutdrucks oder der Körpertemperatur.

Darüberhinaus offenbart die US 2 533 404 ein künstliches Bein mit einem Schaft, an dessen Ende im Inneren eine Druckkammer angeordnet ist, an welche das sich im Schaft befindliche Stumpfende angrenzt. Die Druckkammer verfügt ferner über ein in der Schaftwand angeordnetes herausschraubbares Ventil. Mit Hilfe dieses Ventils lässt sich der Luftdruck in der Druckkammer zur Dämpfung des Stumpfendes einstellen.

Die DE 20 2006 007 460 U1 offenbart einen Protheseninnenschaft, in dem eine Elektrode zur Übertragung von myoelektrischen Signalen an die Hautoberfläche des sich im Schaft befindlichen Körperteils integriert ist. Zusätzlich ist unterhalb des Aufnahmebereichs für das Körperteil ein Ventil im Stumpf angeordnet zum Ablassen der im Innenschaft befindlichen Luft.

Die bisher bekannten Prothesen oder Orthesen weisen entweder lediglich eine dieser zusätzlichen Funktionalitäten auf oder müssen für jeden Patienten individuell mit den gewünschten Funktionalitäten ausgestattet werden. Eine nachträgliche Änderung der Funktionalität der Prothese oder Orthese, insbesondere während diese an ein Körperteil angelegt ist, ist dabei nicht mehr möglich, da die entsprechenden Geräte fest in den Prothesenschaft oder die Orthese eingebaut sind.

Ausgehend hiervon ist es eine Aufgabe der vorliegenden Erfindung eine Prothese oder Orthese bereitzustellen, die eine nachträgliche, lokal wählbare Behandlung eines Amputationsstumpfes bzw. eines Körperteils erlaubt sowie eine einfache nachträgliche ein- oder mehrmalige Änderung der Funktionalität ermöglicht.

Die Aufgabe wird durch eine Prothese oder Orthese nach Anspruch 1 gelöst. Vorteilhafte Ausgestaltungen der erfindungsgemäßen Prothese oder Orthese werden in den abhängigen Ansprüchen 2 bis 4 beschrieben.

Die erfindungsgemäße Prothese oder Orthese weist einen Schaft zur Aufnahme eines Körperteils auf. Der Schaft weist eine einen Körperteilaufnahmebereich begrenzende Wandung auf, wobei ein Teil der Wandung als herausnehmbares Wandstück ausgebildet ist. Das Wandstück weist eine dem Körperteilaufnahmebereich zugewandte Innenseite und eine dem Körperteilaufnahmebereich abgewandte Außenseite auf. Durch das Wandstück ist eine Durchgangsöffnung in der Wandung öffenbar und verschließbar, wobei die Durchgangsöffnung den Körperteilaufnahmebereich mit einem Außenbereich der Prothese oder Orthese verbindet.

Das herausnehmbare Wandstück schafft somit einen lokal wählbaren Zugang zum von einer Prothese aufgenommenen Amputationsstumpf bzw. von einer Orthese aufgenommenen Körperteil, sodass der Amputationsstumpf bzw. das Körperteil behandelt werden kann ohne die Prothese oder Orthese abnehmen zu müssen.

Das Wandstück ist vorzugsweise gemäß einem an das Wandstück angrenzenden Teil der Wandung gewölbt ausgebildet, so dass sich das Wandstück glatt oder formgerecht in die Wandung des Schaftes einfügt. In diesem Fall fügt sich das Wandstück auch optisch in die Wandung ein, was die Optik des Schaftes verbessert. Ferner wird hierdurch eine glatte Schaftoberfläche erzielt, die den Tragekomfort beispielsweise unter Kleidung erhöht. Des Weiteren kann das Wandstück auch flächig, kreisförmig, quadratisch, rechteckig, vieleckig, rund und/oder oval, auch unabhängig von der Wölbung oder Krümmung eines an das Wandstück angrenzenden Teils der Wandung, ausgebildet sein.

Vorzugsweise schließt das Wandstück nahezu oder komplett formschlüssig mit dem an das Wandstück angrenzenden Teil der Wandung ab. Dies verbessert die Optik der Prothese oder Orthese.

Das Wandstück kann ferner eine Dichtung zum luftdichten oder nahezu luftdichten Verschließen der Durchgangsöffnung aufweisen. Auf diese Weise kann nach dem Verschließen der Durchgangsöffnung mit dem Wandstück ein Vakuum im Prothesenschaft aufrecht erhalten werden. Die Dichtung kann auch zur Abdichtung der Durchgangsöffnung gegen Feuchtigkeit und/oder Schmutz dienen. Dies das Eindringen von Schmutz und Feuchtigkeit zwischen Wandstück und Wandung. Die Dichtung kann als ein um das Wandstück umlaufender Dichtring ausgestaltet sein.

Zur Befestigung des Wandstücks an der Wandung kann die Außenseite des Wandstücks Haken und eine dem Körperteilaufnahmebereich abgewandte Außenseite eines an das Wandstück angrenzenden Teils der Wandung Hinterschneidungen oder Ösen aufweisen, durch welche das Wandstück mit der Wandung verklemmbar ist.

Alternativ kann die Wandung ein die Durchgangsöffnung umlaufendes Gewinde aufweisen, in das das Wandstück einschraubbar und aus dem das Wandstück herausschraubbar ist.

Das Wandstück kann eine Dicke aufweisen, die kleiner, größer oder gleich einer Dicke eines an das Wandstück angrenzenden Teils der Wandung ist. Ferner kann das Wandstück eine Breite zwischen 2 und 10 cm, vorzugsweise zwischen 4 und 8 cm, bevorzugt von 6 cm, eine Länge zwischen 2 und 10 cm, vorzugsweise zwischen 4 und 8 cm, bevorzugt von 6 cm, und/oder eine Dicke zwischen 0,5 und 5 cm, vorzugsweise zwischen 1 und 3 cm, besonders bevorzugt von 2 cm aufweisen. Im Falle eines runden Wandstücks kann das Wandstück einen Durchmesser zwischen 2 und 10 cm, vorzugsweise zwischen 4 und 8 cm, bevorzugt von 6 cm aufweisen.

Das Wandstück und die Wandung können gleiche und/oder unterschiedliche Materialien aufweisen oder daraus bestehen.

In einer vorteilhaften Ausgestaltung der Erfindung kann das Wandstück mit einer oder mit mehreren speziellen Funktionalitäten ausgestattet sein. Zum einen kann auf der Innenseite des Wandstücks ein Druckpolster zur Akupressur eines speziellen Akupressurpunktes oder einer Nervenbahn eines Amputationsstumpfes oder eines Körperteils angebracht sein. Ein derartiges Druckpolster kann auch zur Fixierung der Prothese oder Orthese an dem Körperteil dienen. Das Druckpolster enthält bevorzugt Luft, Schaumstoff, eine Pelotte, ein elastisches Material, ein Material mit einem weichen Shore-Grad und/oder ein rigides Material oder besteht daraus. Eine Oberfläche des Druckpolsters kann glatt, rau und/oder adhäsiv sein und/oder ein Relief und/oder Noppen aufweisen. Die Noppen können dabei vollständig mit Material gefüllt oder hohl gestaltet sein.

Auf der Innenseite des Wandstücks ist, wie im unabhängigem Anspruch 1 definiert, ein Medikamentendepot zur Abgabe eines Medikaments an einen Amputationsstumpf oder ein Körperteil angebracht oder in das Wandstück integriert. An einem Wandstück können darüberhinaus auch verschiedene Medikamentendepots angebracht oder in das Wandstück integriert sein, wenn verschiedene Medikamente gleichzeitig verabreicht werden sollen.

Des Weiteren kann auf der Innenseite des Wandstücks ein Wärme-/KühlElement zur Erwärmung und/oder Kühlung des Körperteils und/oder von arteriellem Blut an einer zentralen Einströmstelle des arteriellen Blutes angebracht oder in das Wandstück integriert sein. Hierdurch kann beispielsweise einem oft beklagten Kältegefühl in einem Amputationsstumpf entgegengewirkt werden.

Des Weiteren kann auf der Innenseite des Wandstücks ein eine Feuchtigkeit aufsaugendes Material oder ein elektronisch und/oder mechanisch wirkendes Entfeuchtungselement zur Dehydrierung eines feuchten Innenschaftklimas angebracht oder in das Wandstück integriert sein. Das eine Feuchtigkeit aufsaugende Material kann beispielsweise in Form von Pads am Wandstück angebracht sein.

Ferner ist es denkbar, auf der Innenseite des Wandstücks ein Messgerät zur Messung von Körperzuständen, eines Blutdrucks, einer Strömungsgeschwindigkeit von Blut und/oder einer Körpertemperatur anzubringen oder in das Wandstück zu integrieren.

Darüberhinaus ist für Langzeitdiagnosen die Abfrage von bionischen Daten interessant. Hierzu kann auf der Innenseite des Wandstücks ein Messgerät zur Erfassung von bionischen Daten, wie z. B. eines Oberflächendruckes an definierten Messpunkten, einer Schaftinnentemperatur und/oder eines Feuchtigkeitsgrades angebracht oder in das Wandstück integriert sein.

In einer weiteren vorteilhaften Ausgestaltung der Erfindung kann auf der Innenseite des Wandstücks ein Permanentmagnet oder ein Elektromagnet zur Erzeugung eines Magnetfeldes angebracht oder in das Wandstück integriert sein. Gepulste elektromagnetische Felder (PEMF) sollen bei bestimmten Frequenzen den Organismus bioenergetisch positiv beeinflussen und die Durchblutung fördern, um den Zellstoffwechsel anzuregen.

Eine weitere Möglichkeit sieht vor, auf der Innenseite des Wandstücks ein Piezoelement zur elektrischen und/oder mechanischen Stimulation des Körperteils anzubringen oder in das Wandstück zu integrieren.

Weiterhin kann auf der Innenseite des Wandstücks ein Vibrationselement angebracht oder in das Wandstück integriert sein, wobei das Vibrationselement mit dem Körperteil in mechanischem Kontakt steht. Das Vibrationselement überträgt mechanische Schwingungen auf das von der Prothese oder Orthese aufgenommene Körperteil. Dies fördert die Relaxierung der Muskulatur und die Durchblutung des Körperteils und reduziert Muskelverspannungen.

Ferner kann auf der Innenseite des Wandstücks eine Elektrode zur Abnahme von myoelektrischen Impulsen von dem Körperteil zur Steuerung von mechanischen Gelenken der Prothese oder Orthese angebracht oder in das Wandstück integriert sein.

Zur Fern-Überwachung der Rehabilitationstherapie oder der Tragezeit kann auf der Innenseite des Wandstücks ein Temperatur- und/oder Bewegungslogger angebracht oder in das Wandstück integriert sein.

Eine weitere vorteilhafte Ausgestaltung der Erfindung sieht vor, dass auf der Innenseite des Wandstücks eine TENS-Elektrode oder ein TENS-Gerät angebracht oder in das Wandstück integriert ist, wobei die TENS-Elektrode oder das TENS-Gerät mit dem Körperteil in elektrisch leitendem Kontakt steht. Ein TENS-Gerät dient zur Schmerzbehandlung von Gliedmaßen. Die TENS-Geräte geben über Elektroden einen Reizstrom an die Haut ab. Das Therapieziel ist die Schmerzreduktion durch Verringerung oder Verhinderung der Schmerzweiterleitung an das Gehirn. Ein derartiges TENS-Gerät wird beispielsweise bei der Behandlung des Phantomschmerzes angewandt.

Des Weiteren kann auf der Innenseite des Wandstücks ein Sensor für eine funktionelle Elektrostimulation des Körperteils, z.B. zur Behandlung nach einem Schlaganfall, angebracht oder in das Wandstück integriert sein.

Es ist auch vorteilhaft, wenn die Wandung eine Vielzahl von herausnehmbaren Wandstücken aufweist, wobei durch ein Wandstück jeweils eine Durchgangsöffnung öffenbar und verschließbar ist, wobei die Durchgangsöffnungen den Körperteilaufnahmebereich mit einem Außenbereich der Prothese oder Orthese verbinden. In diesem Fall können mehrere verschiedene Stellen des von der Prothese oder der Orthese aufgenommenen Körperteils von außen zugänglich gemacht werden. Außerdem ermöglicht dies verschiedene Wandstücke mit verschiedenen Funktionalitäten auszustatten.

Ein oder mehrere oder alle Wandstücke können darüberhinaus auch entlang von Akupunktur-Meridianen und/oder Nervenbahnen des Körperteils angeordnet sein. Durch an den Wandstücken angeordnete Druckpolster oder TENS-Elektroden können diese Akupunktur-Meridiane und/oder Nervenbahnen dann stimuliert und Schmerzen im Körperteil behandelt werden.

Ferner können ein oder mehrere oder alle Wandstücke entlang von Bereichen zwischen Muskeln des Körperteils angeordnet sein. Werden die Wandstücke dann beispielsweise mit jeweils einem Druckpolster ausgestattet, kann die Prothese oder Orthese an den Muskellücken beispielsweise gegen ein Verdrehen entlang des Umfangs des Körperteils oder ein Verrutschen in Längsrichtung des Körperteils abgestützt und somit der Sitz der Prothese oder Orthese verbessert werden.

Der Prothesenschaft ist bevorzugt zylinderförmig oder konisch gestaltet und kann einschalig oder auch mehrschalig ausgebildet sein.

Die hier beschriebene Prothese oder Orthese kann beispielsweise eine Arm- oder Beinprothese oder -orthese oder ein Rumpfkorsett sein.

Nachfolgend werden einige vorteilhafte Ausführungsbeispiele einer erfindungsgemäßen Prothese oder Orthese anhand von Figuren genauer beschrieben. Die beschriebenen Merkmale sind jedoch nicht nur in Kombination der offenbarten Ausführungsbeispiele möglich, sondern können auch unabhängig vom konkreten Ausführungsbeispiel in verschiedenen Kombinationen realisiert sein. Ferner bezeichnen gleiche oder ähnlichen in den Figuren verwendete Bezugszeichen gleiche oder ähnliche Merkmale.

Es zeigen
- Figur 1: einen erfindungsgemäßen Prothesenschaft, wobei sich das Wandstück in geöffneter Position befindet, in einer Perspektivansicht,
- Figur 2: den erfindungsgemäßen Prothesenschaft aus Figur 1, wobei sich das Wandstück in geschlossener Position befindet, in einer Perspektivansicht,
- Figur 3: einen erfindungsgemäßen Prothesenschaft mit einem Wandstück in einer perspektivischen Schnittansicht,
- Figur 4: einen erfindungsgemäßen Prothesenschaft mit einem Wandstück in einer weiteren perspektivischen Schnittansicht,
- Figur 5: eine Wandung eines erfindungsgemäßen Prothesenschafts mit einem Grundrahmen für das Wandstück in einer transparenten Ansicht,
- Figur 6: eine perspektivische Schnittansicht eines erfindungsgemäßen Wandstücks und eines dazugehörigen Grundrahmens,
- Figur 7: eine transparente Schnittansicht eines erfindungsgemäßen Wandstücks und eines dazugehörigen Grundrahmens,
- Figur 8: eine Perspektivansicht eines erfindungsgemäßen Wandstücks und
- Figur 9: eine weitere Perspektivansicht eines erfindungsgemäßen Wandstücks.

Es werden die folgenden Bezugszeichen verwendet:
- 1: Wandung
- 2: Wandstück
- 3: Grundrahmen
- 4: Durchgangsöffnung
- 5: Verriegelungsschieber
- 6: Einsatz
- 7: Dichtring
- 8: Überstand
- 9: Hinterschneidung
- 10: Freiraum
- 11: Grundkörper
- 12: Griffe
- 13: Innenseite
- 14: Plättchen
- 15: Öffnung

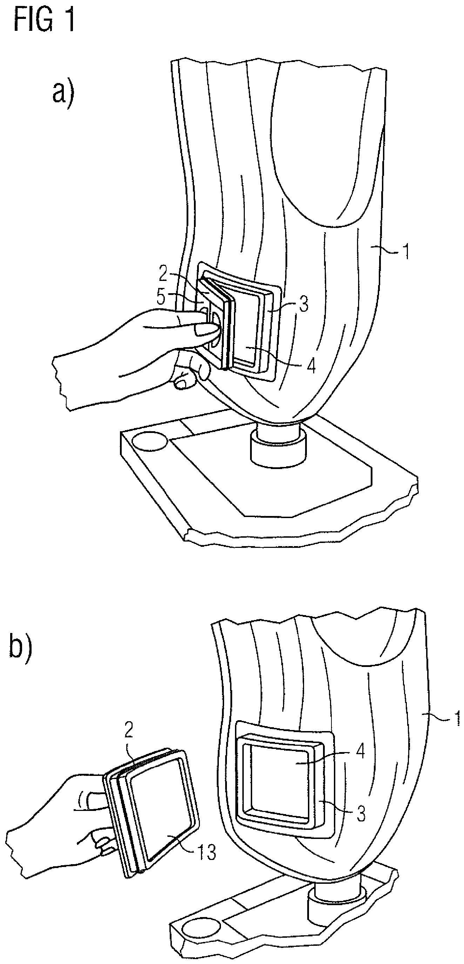

Figur 1a zeigt einen erfindungsgemäßen Prothesenschaft in einer Perspektivansicht. Der Prothesenschaft weist eine Wandung 1 auf, die einen Körperteilaufnahmebereich zur Aufnahme eines Amputationsstumpfes begrenzt. Im unteren, also dem Körper abgewandten Bereich, der Wandung 1 des Prothesenschafts ist ein rechteckiges Wandstück 2 mit abgerundeten Ecken angeordnet. Das Wandstück 2 ist nach außen konvex gewölbt und somit gemäß an das Wandstück 2 angrenzender Bereiche der Wandung 1 gewölbt ausgebildet, so dass sich das Wandstück 2 in einer geschlossenen Position im Wesentlichen glatt und formschlüssig in die Wandung 2 einfügt. Die Durchgangsöffnung 4 ist von einem Grundrahmen 3 umgeben, in den das Wandstück 2 zum Verschließen der Durchgangsöffnung 4 eingesetzt wird. An seiner dem Körperteilaufnahmebereich abgewandten Seite weist das Wandstück einen Verriegelungsschieber 3 auf. Durch Zusammendrücken des Verriegelungsschiebers 3 mit Daumen und Zeigefinger, wie in Figur 1a gezeigt, lässt sich das Wandstück 2 aus dem Grundrahmen 3 entriegeln und aus der Wandung 1 entfernen.

Figur 1a und 1b zeigen das Wandstück 2 in einer geöffneten Position, so dass sich eine Durchgangsöffnung 4 in der Wandung 2 ergibt.

In Figur 1b ist eine dem Körperteilaufnahmebereich zugewandte Innenseite 13 des Wandstücks 2 zu sehen. Die Innenseite 13 ist konkav geformt und somit gemäß einer dem Körperteilaufnahmebereich zugewandten Innenseite der Wandung 1 ebenfalls nach außen gewölbt geformt, so dass sich in einer geschlossenen Position des Wandstücks 2 auch auf der Innenseite der Wandung 1 ein glatter Verlauf zwischen Wandung 1 und Wandstück 2 ergibt.

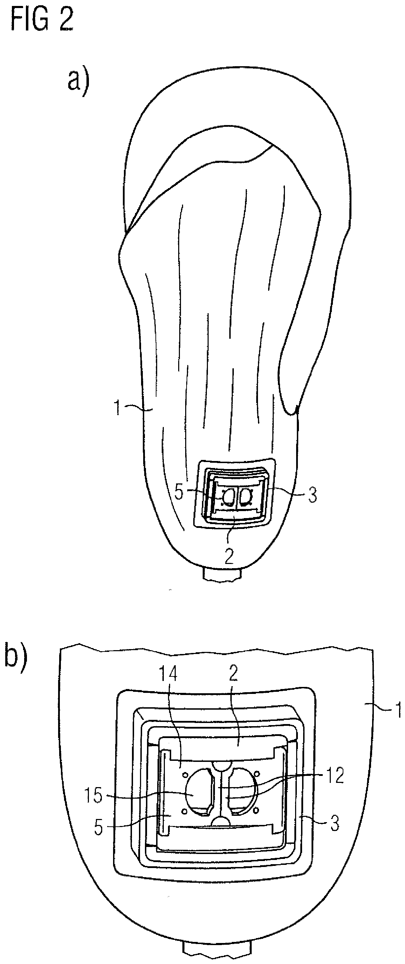

Figur 2a zeigt den Prothesenschaft aus Figur 1a ebenfalls in einer Perspektivansicht, wobei sich das Wandstück 2 nun in einer geschlossenen Position, also eingesetzt in die Wandung 1 des Prothesenschafts, befindet.

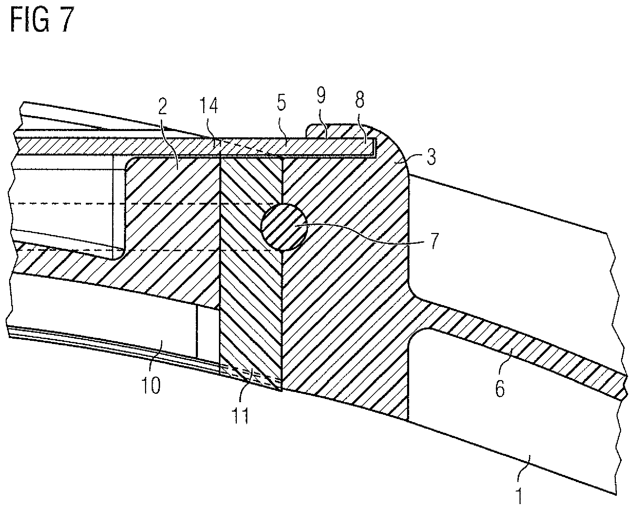

Figur 2b zeigt einen dem Körper abgewandten Bereich des Prothesenschafts wie in Figur 1b, wobei sich das Wandstück 2 in geschlossener Position befindet. Um das Wandstück ist der Grundrahmen 3 in der Wandung 1 angeordnet, der als Aufnahme für das Wandstück 2 dient. Das Wandstück 2 ist mittels des Verriegelungsschiebers 5 am Grundrahmen 3 befestigt. Der Verriegelungsschieber 5 weist zwei gegenüberliegende Plättchen 14 auf, die sich entlang einer Längsachse des rechteckigen Wandstücks 2 zusammenschieben lassen. In die gegenüberliegenden Plättchen 14 ist jeweils eine Öffnung 15 eingelassen, die zum jeweils gegenüberliegenden Plättchen 14 hin von einem stegförmigen Griff 12 begrenzt wird. Mittels der Griffe 12 lassen sich die Plättchen 14 mit Daumen und Zeigefinger zusammenschieben. Zum Grundrahmen 3 hin weisen die Plättchen 14 Überstände 8 auf, die sich senkrecht zur Längsachse des Wandstücks 2 erstrecken. Im auseinandergezogenen Zustand schieben sich die Überstände 8 in den Plättchen 14 benachbarte Hinterschneidungen 9 des Grundrahmens 3 (siehe z.B. Figur 7) und halten so, das Wandstück 2 im Grundrahmen 3.

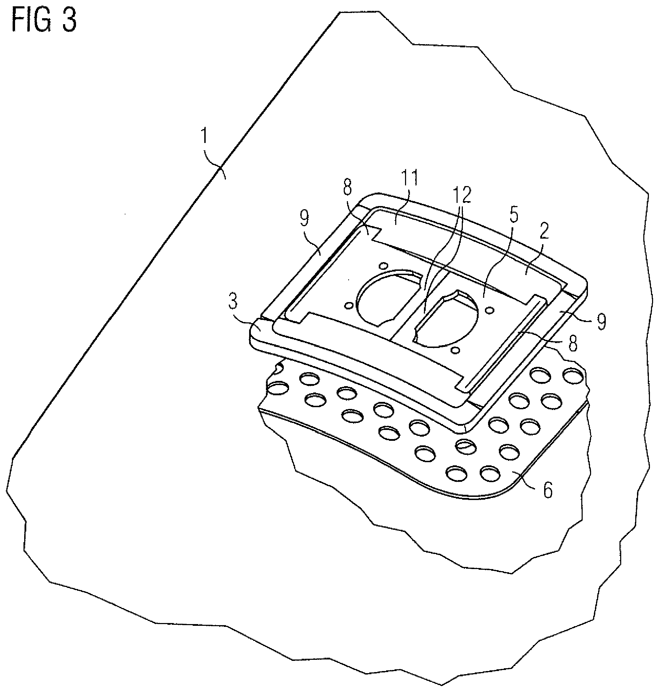

Figur 3 zeigt einen Bereich der Wandung 1 eines Prothesenschafts in einer perspektivischen Schnittansicht. In die Wandung 1 ist das Wandstück 2 eingesetzt. Der Grundrahmen 3 ist in Richtung der Innenseite der Wandung 1 einstückig mit einem flächigen, im Wesentlichen rechteckigen Einsatz 6 verbunden, der in etwa parallel zur Wandung 1 in die Wandung 1 eingelassen ist. Der Einsatz 6 weist ebenfalls die Durchgangsöffnung 4 auf, bildet also ebenfalls eine Begrenzung der Durchgangsöffnung 4. Der Einsatz 6 ragt auf der der Durchgangsöffnung 4 abgewandten Seite über den Grundrahmen 3 hinaus. Ferner weist der Einsatz 6 eine geringere Stärke als die Wandung 1 auf und ist perforiert, um ein Gewicht der Prothese gering zu halten. Der Einsatz 6 dient als Verankerung des Grundrahmens 3. Das Wandstück 2 weist einen umlaufenden Grundkörper 11 auf, auf dem der Verriegelungsschieber 5 angebracht ist.

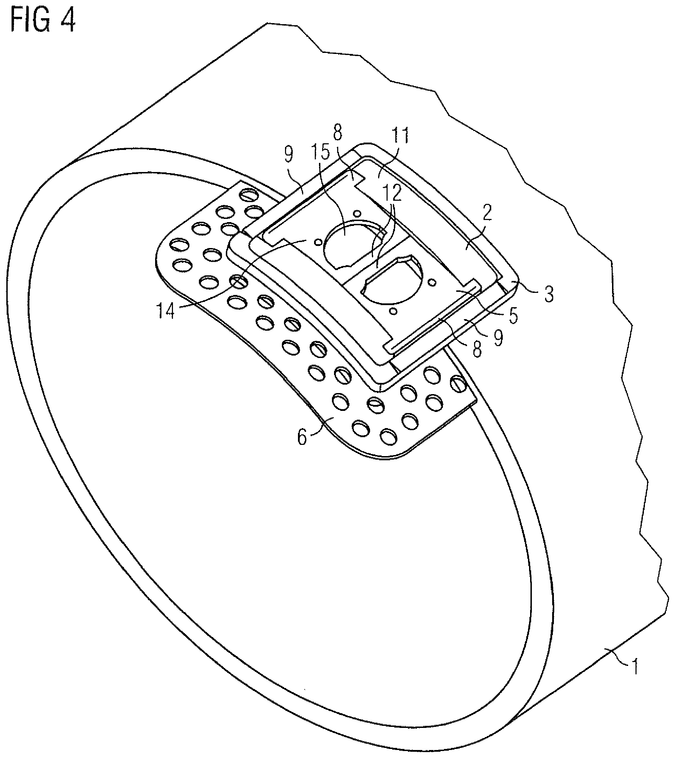

Figur 4 zeigt den Bereich der Wandung 1 eines Prothesenschafts mit einem Wandstück 2 wie in Figur 3 in einer weiteren perspektivischen Schnittansicht mit einem Schnitt durch den Prothesenschaft senkrecht zur Längsachse. Ferner zeigt Figur 3, dass der Einsatz 6 des Grundrahmens 3 parallel zur Wandung 1 in die Wandung 1 eingebettet ist.

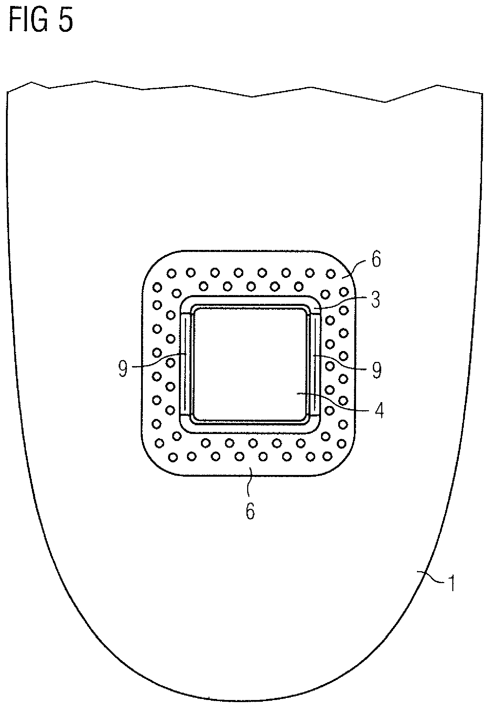

Figur 5 zeigt eine transparente Ansicht der Wandung 1 eines Prothesenschafts mit dem Grundrahmen 3 und der Durchgangsöffnung 4. Der Grundrahmen 3 ist von dem Einsatz 6 umgeben. Auf einer dem Körperteilaufnahmebereich abgewandten Seite weist der Grundrahmen 3 auf Seiten, die in etwa parallel zur Längsachse des Prothesenschafts verlaufen, Hinterschneidungen 9 auf, in welche Plättchen 14 zur Verriegelung eines Wandstücks 2 eingreifen.

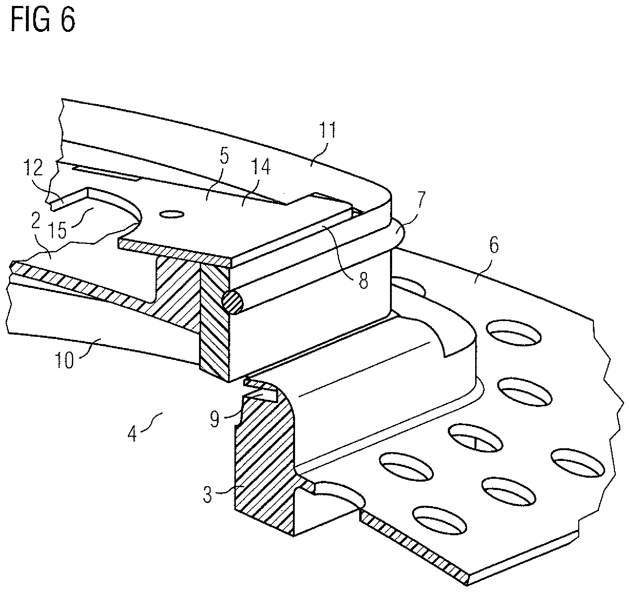

Figur 6 zeigt einen Bereich des Wandstücks 2 sowie des Grundrahmens 3 in einer perspektivischen Schnittansicht, wobei das Wandstück 2 nicht mit dem Grundrahmen 3 verriegelt ist, sondern sich außerhalb des Grundrahmens 3 befindet. Im Vordergrund ist die Hinterschneidung 9 zu sehen, in welche der Überstand 8 im verriegelten Zustand eingreift. Der Grundrahmen 3 geht ferner in eine zur Durchgangsöffnung 4 abgewandte Richtung einstückig in den Einsatz 6 über, der als Verankerung des Grundrahmens 3 in der Wandung 1 dient. Auf einer dem Grundrahmen 3 zugewandten Seite des umlaufenden Grundkörpers 11 des Wandstücks 2 weist das Wandstück 2 einen Dichtring 7 zur weiteren Abdichtung des Bereichs zwischen Wandstück 2 und Grundrahmen 3 zur Aufrechterhaltung eines Vakuums im Prothesenschaft sowie gegen Schmutz und Feuchtigkeit auf. Ferner weist das Wandstück auf einer dem Körperteilaufnahmebereich zugewandten Seite innerhalb des umlaufenden Grundkörpers 11 einen Freiraum 10 auf, der eine Aufnahme von Geräten mit unterschiedlichen Funktionalitäten ermöglicht. Beispielsweise kann in dem Freiraum 10 ein medizinisches Trägerpad mit einem Medikament zur Abgabe an das sich im Körperteilaufnahmebereich befindliche Körperteil angeordnet werden. Ferner ist es möglich im Freiraum 10 ein Druckpolster, eine TENS-Elektrode, ein Vibrationselement, ein eine Feuchtigkeit aufsaugendes Material usw. anzubringen.

Figur 7 zeigt eine transparente Schnittansicht der Wandung 1, in der sich das Wandstück 2 befindet. Das Wandstück 2 ist dabei zur Hälfte abgebildet. Der Verriegelungsschieber 5 befindet sich im verriegelten Zustand. Das Plättchen 14 ist zum Grundrahmen 3 hin verschoben, so dass der Überstand 8 des Plättchens 14 in die Hinterscheidung 9 des Grundrahmens 3 greift. Ferner ist in der Wandung 1 die Verankerung des Grundrahmens 3 mittels des Einsatzes 6 zu sehen, der parallel zu und in etwa in der Mitte der Wandung 1 verläuft.

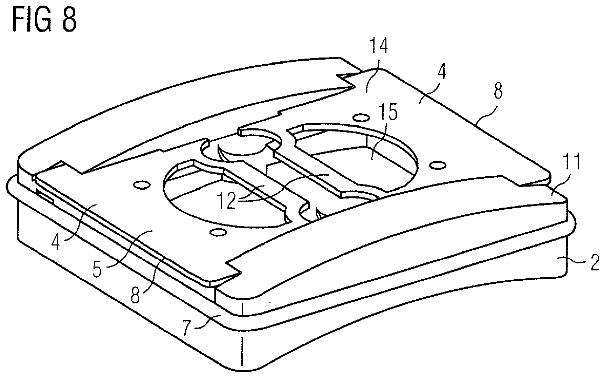

Figur 8 zeigt das Wandstück 2 in einer Perspektivansicht. Das Wandstück 2 weist den umlaufenden Grundkörper 11 auf, der etwa auf halber Höhe einen umlaufenden Dichtring 7 aufweist. Auf der Oberseite des Grundkörpers 11, also auf einer dem Körperteilaufnahmebereich abgewandten Seite, ist der Verriegelungsschieber 5, der die zwei gegeneinander verschiebbaren, flächig ausgebildeten und in etwa parallel zur Wandung liegenden Plättchen 14 aufweist, angeordnet. Im verriegelten Zustand ragen die Überstände 8 der Plättchen 14 über den Grundköper 11 und den Dichtring 7 hinaus, so dass diese in die Hinterschneidungen 9 des Grundrahmens 3 eingreifen können. Hierdurch ergibt sich eine formschlüssige Verbindung zwischen dem Verriegelungsschieber 5 und dem Grundrahmen 3. Werden die Griffe 12 zusammengedrückt, verschieben sich die Plättchen 14 aufeinander zu, und die Überstände 8 werden aus den Hinterscheidungen 9 herausgezogen. Hierdurch löst sich die Verbindung zwischen dem Verriegelungsschieber 5 und dem Grundrahmen 3.

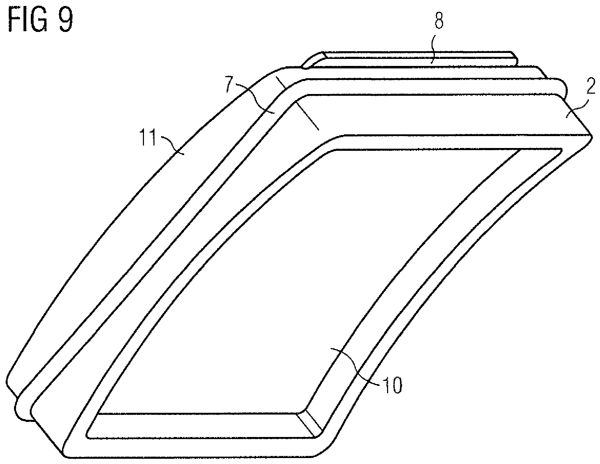

Figur 9 zeigt das Wandstück 2 in einer perspektivischen Unteransicht, das eine dem Körperteilaufnahmebereich zugewandte Seite des Wandstücks 2 zeigt. Innerhalb des umlaufenden Grundkörpers 11 befindet sich der Freiraum 10 zur Aufnahme eines Gerätes, wie z.B. eines medizinischen Trägerpads.

## Patentansprüche

1. Prothese oder Orthese mit einem Schaft zur Aufnahme eines Körperteils, wobei der Schaft eine einen Körperteilaufnahmebereich begrenzende Wandung (1) aufweist,
wobei ein Teil der Wandung (1) als herausnehmbares Wandstück (2) mit einer dem Körperteilaufnahmebereich zugewandten Innenseite (13) und einer dem Körperteilaufnahmebereich abgewandten Außenseite ausgebildet ist, durch welches eine Durchgangsöffnung (4) in der Wandung (1) öffenbar und verschließbar ist, wobei die Durchgangsöffnung (4) den Körperteilaufnahmebereich mit einem Außenbereich der Prothese oder Orthese verbindet, um einen Zugang zu dem Körperteil zur Behandlung des Körperteils zu schaffen, wenn sich der Körperteil in dem Schaft befindet,
**dadurch gekennzeichnet, dass**
auf der Innenseite (13) des Wandstücks (2) ein Medikamentendepot zur Abgabe eines Medikaments an das Körperteil angebracht oder in das Wandstück (2) integriert ist.

2. Prothese oder Orthese nach dem vorhergehenden Anspruch, **dadurch gekennzeichnet, dass** das Wandstück (2) eine Dichtung (7) zum luftdichten Verschließen der Durchgangsöffnung (4) aufweist,

3. Prothese oder Orthese nach einem der vorhergehenden Ansprüche, **dadurch gekennzeichnet, dass** die Außenseite des Wandstücks (2) Haken (8) und eine dem Körperteilaufnahmebereich abgewandte Außenseite eines an das Wandstück (2) angrenzenden Teils der Wandung (1) Hinterschneidungen (9) oder Ösen aufweist, durch welche das Wandstück (2) mit der Wandung (1) verklemmbar ist.

4. Prothese oder Orthese nach einem der vorhergehenden Ansprüche, **dadurch gekennzeichnet, dass** auf der Innenseite (13) des Wandstücks (2) ein Druckpolster zur Akupressur und/oder zur Fixierung der Prothese oder Orthese an dem Körperteil befestigt oder in das Wandstück (2) integriert ist.

## Claims

1. A prosthesis or orthesis having a shaft to receive a body part, wherein the shaft has a wall (1) bounding a body part receiving region, wherein a part of the wall (1) is in the form of a removable wall piece (2), with an inner side (13) facing the body part receiving region and an outer side remote from the body part receiving region, by means of which a through opening (4) in the wall (1) can be opened and closed, wherein the through opening (4) connects the body part receiving region to an outer region of the prosthesis or orthesis in order to create an access to the body part for treatment thereof when the body part is located in the shaft, **characterised in that** on the inner side (13) of the wall piece (2), a medicine store for dispensing a medicine onto the body part is attached or is integrated in the wall piece (2).

2. A prosthesis or orthesis according to the preceding claim, **characterised in that** the wall piece (2) has a sealing means (7) for airtight closure of the through opening (4).

3. A prosthesis or orthesis according to any one of the preceding claims, **characterised in that** the outer side of the wall piece (2) has hooks (8) and an outer side, remote from the body part receiving region, of a part, adjoining the wall piece (2), of the wall (1) has undercuts (9) or eyes, by means of which the wall piece (2) can be clamped to the wall (1).

4. A prosthesis or orthesis according to any one of the preceding claims, **characterised in that** on the inner side (13) of the wall piece (2), a pressure pad for acupressure and/or for fixing the prosthesis or orthesis to the body part is secured to the wall piece (2) or integrated therein.

## Revendications

1. Prothèse ou orthèse avec une tige pour le logement d'une partie du corps, la tige comprenant une paroi (1) limitant une partie de logement de partie du corps,
une partie de la paroi (1) étant conçu comme une portion de paroi amovible (2) avec un côté interne (13) orienté vers la partie de logement de partie du corps et un côté externe opposé à la partie de logement de partie du corps, à l'aide de laquelle une ouverture de passage (4) dans la paroi (1) peut être ouverte ou fermée, l'ouverture de passage (4) reliant la partie de logement de partie du corps avec une partie externe de la prothèse ou de l'orthèse, afin de créer un accès à la partie du corps pour le traitement de la partie du corps, lorsque la partie du corps se trouve dans la tige,
**caractérisée en ce que**
un dépôt de médicaments pour l'administration d'un médicament à la partie du corps est monté sur le côté interne (13) de la portion de paroi (2), ou est intégré dans la portion de paroi (2).

2. Prothèse ou orthèse selon la revendication précédente, **caractérisée en ce que** la portion de paroi (2) comprend un joint d'étanchéité (7) pour la fermeture étanche à l'air de l'ouverture de passage (4).

3. Prothèse ou orthèse selon l'une des revendications précédentes, **caractérisée en ce que** le côté externe de la portion de paroi (2) comprend des crochets (8) et un côté externe, opposé à la partie de logement de partie du corps, d'une partie de la paroi (1) adjacente à la portion de paroi (2), comprend des contre-dépouilles (9) ou des oeillets à l'aide desquels la portion de paroi (2) peut être serrée avec la paroi (1).

4. Prothèse ou orthèse selon l'une des revendications précédentes, **caractérisée en ce qu'**un rembourrage de compression, pour une acupression et/ou pour la fixation de la prothèse ou de l'orthèse à la partie du corps, est fixé sur le côté interne (13) de la portion de paroi (2) ou intégré dans la portion de paroi (2).
